# EUROPEAN PATENT SPECIFICATION

(11) **EP 1 825 860 B1**
(45) Date of publication and mention of the grant of the patent: **09.07.2014**
(21) Application number: 04802573.8
(22) Date of filing: 29.12.2004
(51) Int. Cl.: A61K 36/00

(54) **A PANAXATRIOL SAPONINS COMPOSITION AND ITS PREPARING METHOD AND THE USE**
PANAXATRIOLSAPONINZUSAMMENSETZUNG, VERFAHREN ZU IHRER HERSTELLUNG UND ANWENDUNG
COMPOSITION DE PANAXATRIOL SAPONINS AINSI QUE SON UTILISATION ET UN PROCEDE POUR LA PREPARER

(30) Priority: 09.11.2004 CN 200410090606
(43) Date of publication of application: 29.08.2007
(73) Proprietor: The Pharmaceutical Factory of Chengdu Hoist Inc., Ltd., Chengdu Sichuan 610075 (CN)
(72) Inventor: WAN, Fang, Chengdu, Sichuan 610225 (CN); YUAN, Yuxiang, Tianjin 300193 (CN); LI, Minezing 10 Floor A seat, Huoju High-Rise, Chengdu, Sichuan 610041 (CN)
(74) Representative: Bergmeier, Werner
(86) International application number: PCT/CN2004/001561
(87) International publication number: WO 2006/050641

(56) References cited:
- EP-A- 1 388 344
- WO-A-01/49704
- CN-A- 1 157 720
- CN-A- 1 334 267
- CN-A- 1 412 162
- CN-A- 1 414 009
- CN-A- 1 424 321
- CN-A- 1 491 658
- CN-A- 1 491 659
- CN-A- 1 513 462
- DATABASE EMBASE [Online] ELSEVIER SCIENCE PUBLISHERS, AMSTERDAM, NL; July 2006 (2006-07), CHEN Y ET AL: "Study on process parameters in multi-stage countercurrent extraction by multi-index optimization method" XP002512372 Database accession no. EMB-2006554781 & CHINESE PHARMACEUTICAL JOURNAL 200607 CN, vol. 41, no. 13, July 2006 (2006-07), pages 998-1001, ISSN: 1001-2494
- LI L ET AL: "Detection of saponins in extract of Panax notoginseng by liquid chromatography-electrospray ionisation-mass spectrometry" ANALYTICA CHIMICA ACTA 20050422 NL, vol. 536, no. 1-2, 22 April 2005 (2005-04-22), pages 21-28, XP002512370 ISSN: 0003-2670
- DOUGLAS DUPLER: "NOTOGINSENG ROOT" 2001, GALE GROUP , XP002512371 GALE ENCYCLOPEDIA OF ALTERNATIVE MEDICINE Retrieved from the Internet: URL:http://findarticles.com/p/articles/mi_ g2603/is_0005/ai_2603000555/print?> [retrieved on 2009-01-27]
- PAN X.: 'Extraction process of Notoginseng total saponin' JOURNAL OF MATHEMATICAL MEDICINE vol. 16, no. 5, 30 April 2003, pages 437 - 438, XP008138005
- Extract of the Chinese Pharmacopoeia, published by the Chemical Industry.

## Description

### Field of Techonology

The present invention relates to the field of traditional Chinese Medicine, in particularly, to a purified extract of a Chinese herbal Radix Notoginseng, method for preparing the same and pharmaceutical use of the same.

### Background

Radix Notoginseng (Panax notoginseng (Burk.) F. H. chen) is also called "Tianqi", "Renshen Sanqi", "Tian Sanqi", "Jinbuhuan" , "Xueshen"etal, or Radix Notoginseng, etc. It is a perennial herb belonging to Panax Linn of Araliaceae. Radix Notoginseng is harvested from the third to the seventh years after it is planted, each plantlet grows three petioles, and each petiole develops seven leaf blades, so Radix Notoginseng is named "Sanqi" in China. The stem, the leaf, and the flower of Radix Notoginseng all can be used as medicine, and it is a unique precious traditional Chinese medicine in China. According to related references, Radix Notoginseng has been in use for nearly 600 years and cultivated for nearly 500 years. Radix Notoginseng is famous for the remarkable effects on activating blood circulation to dissipate blood stasis and eliminating swelling and pain, so it is called "jinbuhuan" or "magical herb of the South China".

CN patent application 93105774 (publication No: CN1083819A, publication date: March 16, 1994) discloses a method for preparing a Panax Notoginseng Saponins. CN patent application 01108591 (publication No: CN1334267A, publication date: February 6, 2002) also discloses a method for preparing a Panax Notoginseng Saponins. CN patent application 02144840 (publication No: CN1412162A, publication date: April 23, 2003), 02158727 (publication No: CN1424321A, publication date: June 18, 2003) also respectively discloses a method for extracting a Panax Notoginseng Saponins from the stem and leaf of a Radix Notoginseng.

WO 01/49704 discloses alcohol extracts of corpus radicis and/or radix fibrosa of ginseng plants.

The methods described in the above-mentioned CN patent applications all involve extraction or preparation of Panax Notoginseng Saponins (PNS). The PNS is a kind of dammarane-type Triterpene ginsenosides, and it can be further classified into panaxadiol saponin (PDS) and panaxatriol saponin (PTS) based on the structural features. Owing to the structure difference presented between these two kinds of saponins, their biology actions are also very different from each other. A PDS has a strong direct effect on anti-inflammation and analgesia, while a PTS has a strong effect on anti-platelet aggregation and suppressing the formation of thrombus. But at present, the conventional pharmaceutical preparation made of PNS hardly actions as an effective medicament dedicated to a specific disease. Therefore, separating and purifying effective part containing different components from Radix Notoginseng is required to provide more suitable Radix Notoginseng medicine directed to a specific disease.

### Summary

One objective of the present invention is to provide PTS comprising a Gensennoside Rg1, a Gensennoside Re and a Notogensennoside R1 as main components.

Another objective of the present invention is to provide a method for preparing the PTS.

Further objective of the present invention is to provide a pharmaceutical use of PTS.

Still another objective of the present invention is to provide a use of PTS in treating disease.

In order to realize the above-mentioned purposes, one embodiment of the present invention provides a PTS which is obtained by purifying a Radix Notoginseng and the PTS mainly comprises a Gensennoside Rg1, a Gensennoside Re and a Notogensennoside R1, wherein the content of the Rg1 is 30~70 wt%, preferably is 40~65 wt%, more preferably is 50~65 wt%, the most preferably is 55~65 wt%; the content of the Gensennoside Re is 4~15 wt%, preferably is 4~10 wt%, more preferably is 6~9 wt%; the content of the Notogensennoside R1 is 7~25 wt%, preferably is 8~18 wt%, more preferably is 10~15 wt%; the content sum of the Gensennoside Rg1, the Gensennoside Re and the Notogensennoside R1 is at most 98 wt%.

The molecular formula and the structural formula of the main components of the PTS provided in present invention are shown in the following table 1:

| **Name of component** | **molecular formula** | molecular weight | **structural formula** |
|---|---|---|---|
| Gensennoside Rg1 | C₄₂H₇₂O₁₄ | 801.02 | 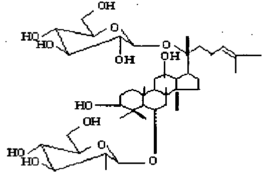 |
| Gensennoside Re | C₄₈H₈₂O₁₈ | 946 | 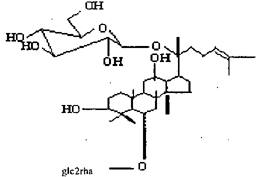 |
| Notogensennoside R1 | C₄₇H₈₀O₁₈ | 933.14 | 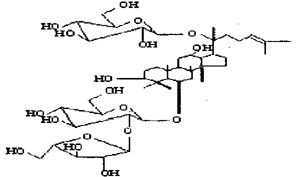 |

The PTS in the above-mentioned embodiment of the present invention can also comprise trace amount of the Gensennoside Rg2, the Gensennoside Rh1, the Notogensennoside R2 and/or the Notogensennoside R3.

The PTS provided in present invention has the following advantages:
(1) In the pharmacodynamics aspect, this PTS composition has more strongly specific effects on patho-physiological process of cardiovascular and cerebrovascular thrombus diseases, it can effectively suppress the formation and development of thrombus, and promote the thrombus dissolution and vascular recanalization;
(2) In the pharmacological action aspect, the specificity of this PTS composition in treating cardiovascular and cerebrovascular thrombus diseases is significant superior to that of the Panax Notoginseng Saponins preparation in the art.

Another embodiment of the present invention provides a method for preparing a PTS, including the following steps:
(1) immersing a Radix Notoginseng with an ethyl alcohol to extract a PTS, wherein the concentration of the ethyl alcohol is 50~90 wt%, preferably is 55~70 wt%, most preferably is about 60 wt%, and the weight of the ethyl alcohol used is at least 3 times of the weight of the Radix Notoginseng, then collecting the ethyl alcohol extracting solution from the Radix Notoginseng;
(2) Adding the ethyl alcohol extracting solution to the chromatographic column of polystyrene-type macroporous absorbing resins, and preferably selecting the nonpolar or weak polar polystyrene-type macroporous absorbing resins for use, wherein the resins including, but are not limited to D101, D140, Dj-1 and D201;
(3) Eluting the chromatographic column of the polystyrene-type macroporous absorbing resins with the ethyl alcohol, wherein the concentration of the ethyl alcohol is 30~50 wt%, preferably is 35~45 wt%, the most preferably is about 40 wt%, then collecting the eluent. The resin column is preferably eluted with water to remove water-soluble impurity before eluting with the ethyl alcohol.

The about 60 wt% ethyl alcohol concentration used in step (1) of the above-mentioned embodiment of the present invention means the concentration is within the acceptable error range, generally 60±3 wt%. The about 40 wt% ethyl alcohol concentration used in step (3) of above-mentioned embodiment of the present invention means the concentration is within the acceptable error range, generally 40±3 wt%.

In the above-mentioned steps (1) and (3), the amount of the ethyl alcohol for use is not particularly limited. But as for step (1), the weight of the ethyl alcohol for use is generally 3~15 times heavier than that of the Radix Notoginseng. If the ethyl alcohol amount to be used is too little, the immersing extraction is not adequate, but if the excess amount of ethyl alcohol is used, it will result in a waste of the ethyl alcohol and increase the energy cost of the subsequent recovering step of the ethyl alcohol. Similarly, as for step (3), the weight of the ethyl alcohol for use is generally 1~15 times heavier than that of the Radix Notoginseng. If the ethyl alcohol amount to be used is too little, the eluting is not adequate, but if the excess amount of ethyl alcohol is used, it will result in a waste of the ethyl alcohol and increase the energy cost of the subsequent recovering step of the ethyl alcohol.

In order to improve the efficiency of the extracting, in consideration of the efficiency of the subsequent steps, pulverizing the Radix Notoginseng is required before the immersing extraction with the ethyl alcohol according to the preparation method described in the step (1) of the present invention is applied. Preferably and the pulverizing method is not limited.

Therefore, the preferred implementation of the step (1) in the method for preparing PTS according to the present invention is as follows:
(a) Pulverizing the Radix Notoginseng to obtain the Radix Notoginseng powder;
(b) Immersing the Radix Notoginseng powder obtained in step (a) with an ethyl alcohol , wherein the weight of the ethyl alcohol is at least 2 times heavier than that of the Radix Notoginseng, and the concentration of the ethyl alcohol is 50-90 wt%, preferably is 55-70 wt%, the most preferably is about 60 wt%; and
(c) Percolating the Radix Notoginseng powder obtained in step (b) with an ethyl alcohol, the weight of the ethyl alcohol is at least 1 time heavier than that of the Radix Notoginseng, and the concentration of the ethyl alcohol is 50-90 wt%, preferably is 55~70 wt%, the most preferably is about 60 wt%, and then collecting the percolating liquid.

The approximate 60 wt% ethyl alcohol concentration used in steps (b) and (c) of above-mentioned embodiment of the present invention means the concentration is within the acceptable error range, generally 60±3 wt%.

The amount of the ethyl alcohol for immersing and percolating the Radix Notoginseng powder in above-mentioned steps (b) and (c) is not particularly limited. But the reasonable weight of the ethyl alcohol for immersing the Radix Notoginseng powder is usually 2-4 times heavier than that of the Radix Notoginseng; the reasonable weight of the ethyl alcohol for percolating the Radix Notoginseng powder is usually 1~15 times heavier than that of the Radix Notoginseng. If the amount of the ethyl alcohol used for immersing and percolating the Radix Notoginseng powder is below the range, the active component in the Radix Notoginseng powder is not extracted efficiently and adequately, but if the amount of the ethyl alcohol used is above the highest limit, extracting efficient can not be improved, and it will result in a waste of the ethyl alcohol and increase the energy cost of the subsequent recovering step of the ethyl alcohol.

If necessary, the eluent collected in the step (3) can be further concentrated to obtain ethyl alcohol extracts of PTS, and then drying the ethyl alcohol extracts of PTS. Any common method can be used in the concentration step, but a decompressing concentration method is preferably used. Various drying methods can be used in the drying step, such as a spray drying, a decompressing drying, etc.

In order to obtain a PTS with a higher content of the active components, the eluent collected in step (3) and the concentrated product or the concentrated dried product of the eluent are further purified finely. Various methods can be used for the finely purifying process, including but not limited to the column chromatography, the solvent extraction, etc. When the column chromatography method is applied to the purification, the weakly basic anion exchange resin is preferably used, including but not limited to BS-II, D301, and D313. When the solvent extraction method is applied, the solvents that can be used include a butanol or an ethyl acetate, etc.

In order to reduce the cost, ethyl alcohol collected from the ethyl alcohol extracting of step (1) the percolating liquid of step (c) and the eluent of step (3) can be recovered in the method for preparing a PTS of the invention, and a decompressing recovering method is preferably used.

The method for preparing a PTS provided in the invention can effectively separate a PTS from a Panax Notoginseng, moreover, the solvent used in the method is safe, easily available, free of organic residual, and recoverable, so the preparation cost is greatly reduced.

Another embodiment of the invention provides pharmaceutical use of PTS in preparing medicine for treating vascular thrombus diseases, especially for treating cardiovascular and cerebrovascular thrombus diseases. The Cardiovascular and cerebrovascular thrombus diseases include stroke, hemiplegia, deviation of mouth and tongue, language disorder, unilateral numbness, coronary heart disease, angina, myocardial infarction, retinal vein occlusion, etc.

The dosage forms of PTS provided in the invention include various forms, such as granules, the mixture, the tablet, the medicinal powder, the honey pill, the capsule, the injection, the drops, and the patch, etc. The method and the excipient for preparing these dosage-forms are well known to those skilled in the art, for example, seeing "Traditional Chinese Medicine Pharmaceutics" (published by Shanghai Science and technology Publishing House, November, 1986), "Manual of Medicinal Supplementary Material" (published by Sichuan Science and Technology Publishing House, January, 1995), and Chinese Pharmacopoeia (published by Chemical Industry Publishing House, January, 2000), etc.

The content of PTS in single dosage of the medicine of the invention is not specifically limited, for example, it may be from 50 to 500 milligrams per dosage. Also the administration route of the medicine preparations of the invention is not specifically limited, for example, it may be through an oral administration, an intragastric administration, an intramuscular injection, an intravascular injection, a transdermal administration, a duodenal administration, a pernasal administration, etc.

, The above-mentioned preparation of the invention is highly effective in activating blood circulation to dissipate blood stasis, preventing platelet coagulation and thrombus formation, improving microcirculation, reducing blood viscosity and enhancing blood flow of carotid arteries, so it has a primary use in treating disorders related to cardiovascular and cerebrovascular thrombus diseases, for example, stroke, hemiplegia, deviation of mouth and tongue, language disorder and unilateral numbness.

The invention also provides one therapeutic method for vascular thrombus diseases, characterized in employing effective dosage of PTS to patients. The vascular thrombus diseases includes, but not limited to cardiovascular and cerebrovascular thrombus diseases, for example, stroke, hemiplegia, deviation of mouth and tongue, language disorder, and unilateral numbness, etc. The patients include human, as well as animals such as cat, dog, orangutan and monkey, etc. The effective dosage can vary along with the disease severity degree, the body weight, the body constitution, the age and the case history of the patients, etc. But, doctors or the pharmacists are able to determine appropriate dosage for different patients to take, for example, 1~15mg/ body weight (kg) /day, preferably 2~10mg/ body weight (kg)/day.

### Detailed Description of the Best Embodiments

### Example 1

An omnipotent pulverizer (commercial available from the Shanghai Tianhe Pharmaceutical Machine Factory, model GF-300) is used to pulverize 150 kg of dry rhizome of the Radix Notoginseng (area of production: Wenshan state of Yunnan Province) until the particle size of the coarse powder of the Radix Notoginseng is within the range of 10~24 meshes. The coarse powder of the Radix Notoginseng is filled into a percolating barrel after being immersed with the 60 wt% ethyl alcohol, wherein the weight of the alcoho is 2 times heavier than that of the Radix Notoginseng. Then the coarse powder is percolated with the 60 wt% ethyl alcohol, wherein the weight of the ethyl alcohol is 15 times heavier than that of the Radix Notoginseng. The extracts of the Panax Notoginseng Saponins is obtained after the ethyl alcohol of the percolating fluid is decompressed and recovered. The extracts of Panax Notoginseng Saponins are prepared with purified water to obtain a solution, where the weight of the purified water is 10 times heavier than that of the Radix Notoginseng. The solution is added to the chromatographic column of polystyrene-type macroporous absorbing resins D140 (commercial available from Chengdu Chengguang Chemical Industry Research Institute), and the column is eluted with the purified water, wherein the weight of the purified water is 5 times heavier than that of the Radix Notoginseng. Then the column is eluted with the 40 wt% ethyl alcohol, wherein the weight of the ethyl alcohol is 9 times heavier than that of the Radix Notoginseng, and elutes are collected. Elutes are decompressed and concentrated to recover the ethyl alcohol extracts. Drying the obtained extracts by spray drying, then a 6 kg PTS powder is obtained.

The detection is applied by HPLC on the condition that filler was octadecyl-silyl, the mobile was acetonitrile-water (the volume ratio is 19.5:80.5) and wavelength was 210nm and the result show that the content of the Rg1 in the PTS of Example 1 is 60 wt%, the R1 is 12 wt%, and the Re is 7 wt%.

### Examples 2~4

The same method and step is used in correspondence with Example 1, with the exception that the different concentration and different weight of the ethyl alcohol is applied to immerse, percolate and elute the Radix Notoginseng, and the results obtained are shown in table 2 as follows:

**Table 2**

| Example Number | Weight of the used Radix Notoginseng | Concentration and Weight of the Ethyl alcohol Used for Immersing and Percolating | Concentration and Weight of the Ethyl alcohol Used for Resin and Eluting Resin | Weight of PTS And Content of the Rg1 ,Re and R1 in PTS |
|---|---|---|---|---|
| Ex.2 | 1 kg | 75 wt% ethyl alcohol for immersing, and the weight of the ethyl alcohol is 2 times heavier than that of the Radix Notoginseng | D101 (commercial available from Tianjing Pesticide Plant) | Weiht of the PTS: 0.0735 kg |
| | | 75 wt% ethyl alcohol for percolating, and the weight of the ethyl alcohol is 9 times heavier than that of the Radix Notoginseng | 50 wt%I ethyl alcohol, and 2 times heavier weight of the Radix Notoginseng | Rg1: 35.01 wt% |
| | | | | Re: 4.445 wt% |
| | | | | R1: 11.56 wt% |
| Ex.3 | 1 kg | 60 wt% ethyl alcohol for immersing, and the weight of the ethyl alcohol is 2 times heavier than that of the Radix Notoginseng | D101 (commercial available from Tianjing Pesticide Plant) | Weight of the PTS: 0.0556 kg |
| | | 60 wt% ethyl alcohol for percolating, and the weight of the ethyl alcohol is 9 times heavier than that of the Radix Notoginseng | 40 wt%I ethyl alcohol, and 1.5 times heavier weight of the Radix Notoginseng | Rg1: 60.72 wt% |
| | | | | Re: 7.535 wt% |
| | | | | R1: 12.23 wt% |
| Ex.4 | 1 kg | 60 wt% ethyl alcohol for immersing, and the weight of the ethyl alcohol is 2 times heavier than that of the Radix Notoginseng | D140(commercial available from Chengdu Chengguang chemical industry research institute) | Weight of the PTS: 0.0184 kg |
| | | 60 wt% ethyl alcohol for percolating, and the weight of the ethyl alcohol is 9 times heavier than that of the Radix Notoginseng | 30 wt%I ethyl alcohol, and 1.5 times heavier weight of the Radix Notoginseng | Rg1: 47.415 wt% |
| | | | | Re: 6.505 wt% |
| | | | | R1: 16.15 wt% |

### Example 5

The dried 100g PTS obtained in Example 1 is prepared to a 2.5 L solution with a pH6.0 acetate buffer. The solution is added to the column of weakly basic anion exchange resin BS-II (commercial available from Chengdu Chengguang Chemical Industry Research Institute), wherein the resin column volume is 6L. The column is eluted with a 12L acetate buffer, wherein the buffer pH is 6.0 and the flowing rate is about 0.4 time of the column volume. The eluents are collected and then dried to obtain a 60g fined PTS powder.

The detection is applied by HPLC on the condition that filler was octadecylsilyl, the mobile was acetonitrile-water (the volume ratio is 19.5:80.5) and wavelength was 210nm and the result show that the content of the Rg1 in the PTS of Example 5 is 65 wt%, the R1 is about 20 wt%, the Re is about 8 wt%, the water is about 5 wt%, and the additional impurity is about 2wt%.

### Example 6

The dried 100g PTS obtained in Example 1 is dissolved in the 50 wt% ethyl alcohol, and adding a 100g starch to therein until the mixture is uniformity. Then, the mixture is backfilled into 1000 capsules to granulate it with one-step-granulating method.

### Experimental Example 1 Effects on the accumulation and the release function of the blood platelet

An in vivo experiment is carried out by the turbidimetric method using the PAM-3 double channel blood platelet accumulation meter (commercial available from Danyang Radio Plant of Jiangsu Province) to evaluate the PTS effects on suppressing blood platelet accumulation. The healthy male SD rats were divided into three dosage groups: high (150 mg/kg), middle (75 mg/kg), and low (37.5 mg/kg). The PTS powder obtained in Example 1 is dissolved in saline to prepare a PTS solution. According to the predetermined dosage, the PTS solution is administered through duodenum for one time. 1 hour later, the blood is sampled from the abdominal aorta to prepare a rich platelet plasma, and 3×10⁻⁶ mol/L revulsant ADP is added therein. The experimental results indicate that the PTS prepared in Example 1 can significantly suppress the ADP-induced platelet aggregation of the rats in a dosage-dependant manner. The function intensity of the PTS is basically the same as aspirin.

An in vitro experiment is carried out by the turbidimetric method using the PAM-3 double channel blood platelet accumulation meter (commercial available from Danyang Radio Plant of Jiangsu Province) to evaluate the PTS effects on suppressing blood platelet accumulation. The PTS powder obtained in Example 1 is firstly dissolved in saline to prepare a PTS solution, and the concentration of the PTS solution are respectively 1, 2, and 4 mg/ml. The blood is sampled from the carotid artery of the healthy male rabbits to prepare a rich platelet plasma. The plasma is diluted with the same volume PTS saline solutions. Then 3×10⁻⁶ mol/L revulsant ADP is added into the diluted plasma. The experimental results indicate that the PTS obtained in Example 1 can significantly suppress the ADP-induced platelet aggregation in a dosage-dependant manner and the results are similar to the in vivo experiment of aspirin.

### Experimental Example 2 Influence on the experimental thrombosis model in rats

42 healthy male SD rats (over 300g body weight) are divided into 2 control groups and 4 experimental groups, and each group has 7 SD rats. The PTS powder obtained in Example 1 is dissolved in saline to prepare a PTS solution. In the 4 experimental groups, the PTS saline solution is administered through the duodenum for one time by the dosage of 25 mg/kg, 50 mg/kg, 100 mg/kg and 200 mg/kg respectively. In the negative control group, distilled water is administered through the duodenum by the same volume as the maximum dosage group for one time, while in the positive control group, an aspirin (300 mg/kg) is intragastricly administered for one time. 1 hour later, the thrombosis models in rats are established by using the method of arteriovenous bypass thrombosis. The results prove that the PTS (50 mg/kg, 100 mg/kg, and 200 mg/kg) obtained in Example 1 can significantly suppress the experimental thrombosis in rats. And the higher the dosage, the stronger the effect is.

### Experimental Example 3 Effects on bleeding time in mice

40 healthy male mice of Kunming strain are divided into 1 control group and 3 experimental groups, and each group has 10 mice. In the control group, an aspirin (150 mg/kg) is administered via an intraperitoneal injection for one time. In the 3 experimental groups, the PTS saline solution (50 mg/ml) is administered via an intraperitoneal injection for one time by the dosage of 300 mg/kg, 150 mg/kg, 75 mg/kg respectively. 20 minutes later, cut the peaked part of the mice's tails and collect the blood drop with a filter paper once for 30 seconds until the bleeding stops, and record the bleeding time. The results show that the PTS obtained in Example 1 and the aspirin can both significantly prolong bleeding time and there is no significant difference between them.

### Experimental Example 4 Effects on experimental cerebral ischemia in gerbils

50 healthy gerbils are randomly divided into 1 normal control group, 1 ischemic control group, 1 positive control group, 2 experimental groups, and each group has 10 gerbils. In the normal group, the gerbils are bred in a normal way without any medicine. In other groups, a hemi-cerebral infarction is induced by a unilaterally common carotid artery ligation. In the 2 experimental groups, the PTS saline solution (50mg/ml)prepared in the example 1 is administered via an intraperitoneal injection by the dosage of 150 mg/kg and 75mg/kg respectively once daily for 10 days. In the ischemic control group, a saline solution is administerd via an intraperitoneal injection by the dosage of the same volume as the maximum dosage group once daily for 10 days. In the positive control group, a nimodipine (60 mg/kg) is intragastricly administered once daily for 10 days. 1 hour after the last administration, observing the effects of the PTS obtained in Example 1 on the brain - dysfunction and the changes of Na⁺ and K⁺ induced by cerebral infarction. According to the methods of Butterfield and others (in the National Coronary Disease Symposium collection of 1973, Page 261, published by People's Medical Publishing House in 1974), the animal's behaviors are graded. The result indicates that the PTS obtained in Example 1 can distinctly improve animals' behavior disorder induced by ischemia, so can the positive medicine nimodipine. The contents of Na⁺ and K⁺ in the brain tissue are quantitatively analyzed by a flame emission photometric method. The result indicates that the content of Na⁺ increases while the content of K⁺ reduces in the ischemic cerebral hemisphere. The content changes of Na⁺ and K⁺ are not obvious in the PTS (150 mg/kg) group. The content of K⁺ is reduced while the content of Na⁺ is not changed in the PTS (75 mg/kg) group, and the similar effect is also observed in the positive medicine nimodipine group.

To sum up, the PTS obtained in Example 1 has certain protective function on the experimental cerebral ischemia in gerbils.

### Experimental Example 5 The protective function of PTS on the cerebral infarction induced by blocking middle cerebral artery

### (1) Preventive medicine

50 healthy male Wistar rats are randomly divided into 1 ischemic control group, 1 positive control group, 3 experimental groups, and each group has 10 rats. The PTS powder obtained in Example 1 is dissolved in saline to prepare a PTS suspension. In the 3 experimental groups, the PTS saline solution is intragastricly administered by the dosage of 200 mg/kg, 100mg/kg and 50 mg/kg respectively once daily for 7 days. In the positive control group, a nimodipine (50 mg/kg) is intragastricly administered once daily for 7 days. In the ischemic control group, a distilled water (4 ml/kg) is administered intragastricly once daily for 7 days. In each group, 2 hours after the last administration, a chloral hydrate is administered via an intraperitoneal injection by the dosage of 350 mg/kg to anesthetize the rats. A cerebrum median artery is permanently occluded by burning to cause a local cerebral infarction. Then observing the effects of the PTS obtained in Example 1 as a preventive medicine on the brain - dysfunction and the infarct size of local cerebral infarction. The result indicates that the PTS obtained in Example 1 by the dosage of 200 mg/kg, 100 mg/kg and 50 mg/kg once daily for 7 days has significantly protective effect on local cerebral infarction induced by occluding cerebrum median artery, and the infarct size reduces by 54.2%. By a behavior grading method, the results show that the PTS (200 mg/kg and 100mg/kg) can significantly ameliorate the behavior disorders of the experimental rats, which is similar to the nimodipine.

### (2) Therapeutic medicine

According to body weight, 50 healthy male Wistar rats are randomly divided into 1 ischemic control group, 1 positive control group, 3 experimental groups, and each group has 10 rats. In each group, a chloral hydrate is administered via an intraperitoneal injection by the dosage of 350 mg/kg to anesthetize the rats. A cerebrum median artery is permanently occluded by burning to cause the local cerebral infarction. The PTS powder obtained in Example 1 is dissolved in saline to prepare a PTS suspension. In the 3 experimental groups, the PTS saline solution is intragastricly administered by the dosage of 200 mg/kg, 100mg/kg and 50 mg/kg respectively once daily for 6 days. In the positive control group, a nimodipine (50 mg/kg) is intragastricly administered once daily for 6 days. In the ischemic control group, a distilled water (4 ml/kg) is administered intragastricly once daily for 6 days. 2 hours after the last administration, observing the effects of the PTS obtained in Example 1 as a treatment medicine on the brain - dysfunction and the infarct size of local cerebral infarction. The result indicates that the PTS obtained in Example 1 by the dosage of 200 mg/kg and 100mg/kg once daily for 6 days has an significantly curative effect on the local cerebral infarction induced by occluding cerebrum median artery, and can significantly ameliorate the behavior disorders of experimental rats. The infarct size reduces by 37.1 %.

To sum up, the PTS obtained in Example 1 has a protective function on the local cerebral infarction in rats.

### Experimental Example 6 Influence on blood viscosity in rats

50 healthy male Wistar rats are randomly divided into 1 negative control group, 1 positive control group, 3 experimental groups, and each group has 10 rats. The PTS powder obtained in Example 1 is dissolved in saline to prepare a PTS saline solution. In the 3 experimental groups, the PTS saline solution is administered via an intraperitoneal injection by the dosage of 80 mg/kg, 40mg/kg and 20 mg/kg respectively once daily for 10 days. In the positive control group, a Calan (30 mg/kg) is intraperitonealy injected once daily for 10 days. In the negative control group, a distilled water is administered intragastricly by the dosage of the same volume as the maximum dosage group once daily for 10 days. 1 hour after the last administration, in each group, a pentobarbital sodium is administered via an intraperitoneal injection by the dosage of 40 mg/kg to anesthetize the rats. The blood is sampled from the abdominal aorta, and the blood samples is anticoagulated by a 3.8%sodium citrate solution to assay the blood viscosity in rats by the blood viscosity measure method.

The experimental result indicates that the PTS obtained in the Example 1 that is administered repeatedly by an intraperitoneal injection can significantly reduce the blood viscosity, and the results have statistic difference (p<0.05) in the high dosage group, so does the positive control group. The PTS in the high dosage group also significantly reduces the hematocrit of the blood, but has no influence on blood sedimentation and relative plasma viscosity.

### Experimental Example 7 An acute toxicity experiment

50 healthy NIH mice (18~22 gram body weight) are randomly divided into 5 groups, each of which has10 mice (half male and half female). The medicine dosage in each group is respectively 4444.44 mg/kg, 4000.00 mg/kg, 3600.00 mg/kg, 3240.00 mg/kg, and 2916.00 mg/kg (the ratio of high dosage to low dosage is 1:0.9). The PTS powder obtained in Example 1 is dissolved in sterilized saline to prepare a PTS saline solution. According to the predetermined dosage, the PTS solution is administered via an intravenous injection for one time. According to the experimental result, LD50 (Half Lethal Dosage: the dosage causing the half number of experimental animals to death in the acute toxic experiment) is calculated to be 3422.2 mg/kg (3261.8~3590.6). Before the death, transient convulsions, jumps and respiratory inhibitions emerge in the mice.

### Experimental Example 8 Chronic toxicity experiment

The PTS powder obtained in Example 1 is dissolved in saline to prepare a concentrated PTS suspension. The PTS suspension is intragastricly administered to rats by the dosage of 2 g/kg once daily for 90 days. The physiological condition of rats is evaluated after the experiment. The result shows that there's no abnormality in the general state, the blood and biochemical indexes, the organ coefficient and organ pathology.

Although the invention has been explained with reference to the preferred examples, the invention is not limited to the above-mentioned examples. It should be understood that one person skilled in the art can make various amendments and improvement without departing the scope of the invention, and the scope of the present invention can be defined by the claims.

## Claims

1. A panaxatriol saponins composition obtained by extracting and purifying the Radix Notoginseng, basically comprising a Gensennoside Rg1, a Gensennoside Re and a Notogensennoside R1, wherein the content of the Gensennoside Rg1 is 30~70 wt%, the Gensennoside Re is 4~15 wt%, the Notogensennoside R1 is 7-25 wt%, and the maximum content sum of the Gensennoside Rg1, the Re and the Notogensennoside R1 is 98 wt%.

2. The Panaxatriol saponins composition according to claim 1, characterized that the content of the Gensennoside Rg1 is 40-65 wt%, the Gensennoside Re is 4~0 wt%, and the Notogensennoside R1 is 8~18 wt%.

3. The Panaxatriol saponins composition according to claim 2, characterized that the content of the Gensennoside Rg1 is 50-65 wt%, the Gensennoside Re is 6~9 wt%, and the Notogensennoside R1 is 10~15 wt%.

4. The Panaxatriol saponins composition according to claim 3, characterized that the content of the Gensennoside Rg1 is 55-65 wt%.

5. The Panaxatriol saponins composition according to the any one of claims 1-4, wherein the dosage forms of the panaxatriol saponins composition include the granules, the mixture, the tablet, the medicinal powder, the honey pill, the capsule, the injection, the drops or the patch.

6. A method for preparing a Panaxatriol saponins composition as specified in claim 1, comprising the following steps:
(1) immersing a Radix Notoginseng with a 50-90 wt% ethyl alcohol to extract a panaxatriol saponins composition , wherein the weight of the ethyl alcohol used is at least 3 times heavier than that of the Radix Notoginseng, then collecting the ethyl alcohol extracting solution of the Radix Notoginseng;
(2) adding the ethyl alcohol extracting solution to a chromatographic column of polystyrene-type macroporous absorbing resins; and
(3) eluting the chromatographic column of the polystyrene-type macroporous absorbing resins with a 30-50 wt% ethyl alcohol and collecting the eluent.

7. The method for preparing a Panaxatriol saponins composition according to claim 6, **characterized in that** the step (1) is completed as follows:
(a) pulverizing the Radix Notoginseng to obtain the Radix Notoginseng powder;
(b) immersing the Radix Notoginseng powder obtained in step (a) with a 50-90 wt% ethyl alcohol, wherein the weight of the ethyl alcohol used is at least 2 times heavier than that of the Radix Notoginseng powder; and
(c) percolating the Radix Notoginseng powder obtained in step (b) with a 50~90 wt% ethyl alcohol, wherein the weight of the ethyl alcohol used is at least 1 time heavier than that of the Radix Notoginseng powder; then collecting the percolating liquid.

8. The method for preparing a Panaxatriol saponins composition according to claim 7, **characterized in that** the concentration of the ethyl alcohol used in steps (b) and (c) is 55-70 wt%.

9. The method for preparing a Panaxatriol saponins composition according to claim 8, **characterized in that** the concentration of the ethyl alcohol used in steps (b) and (c) is about 60 wt%.

10. The method for preparing a Panaxatriol saponins composition according to claim 6, **characterized in that** the concentration of the ethyl alcohol used in step (3) to elute resin is 35-45 wt%, wherein weight of the ethyl alcohol for use is at least 1 time heavier than that the Radix Notoginseng powder.

11. The method for preparing a Panaxatriol saponins composition according to claim 10, **characterized in that** the concentration of the ethyl alcohol used in step (3) to elute resin is about 40 wt%.

12. The method for preparing a Panaxatriol saponins composition according to the any one of claims 6-11, **characterized in that** it further includes the following step: concentrating the eluent collected in step (3) to obtain the panaxatriol saponins composition extracts.

13. The method for preparing a Panaxatriol saponins composition according to the any one of claims 6-11, **characterized in that** it further includes the following step: purifying the eluent obtained in step (3) by column chromatography or solvent extraction.

14. The method for preparing a Panaxatriol saponins composition according to claim 12, **characterized in that** it further comprises the step of drying the panaxatriol saponins extracts.

15. A use of the Panaxatriol saponins composition according to the any one of claims 1-4 in preparing the medicine for treating the vascular thrombus disease.

16. Panaxatriol saponins composition according to claim 1 to 4 for use in a method of treating vascular thrombus disease, wherin said method comprises the administration of an effective dosage of said Panaxatriol saponins composition to patients suffering from vascular thrombus disease.

## Patentansprüche

1. Panaxatriol-Saponine-Zusammensetzung, erhalten durch Extrahieren und Aufbereiten der Radix Notoginseng, hauptsächlich enthaltend ein Ginsenosid Rg1, ein Ginsenosid Re und ein Notoginsenosid R1, wobei der Gehalt an dem Ginsenosid Rg1 30-70 Gew.-%, an dem Ginsenosid Re 4~15 Gew.-%, an dem Notoginsenosid R1 7-25 Gew.-% und die maximale Gehaltssumme des Ginsenosids Rg1, des Re und des Notoginsenosids R1 98 Gew.-% beträgt.

2. Panaxatriol-Saponine-Zusammensetzung nach Anspruch 1, **dadurch gekennzeichnet, dass** der Gehalt an dem Ginsenosid Rg1 40~65 Gew.-%, an dem Ginsenosid Re 4~10 Gew.-% und an dem Notoginsenosid R1 8~18 Gew.-% beträgt.

3. Panaxatriol-Saponine-Zusammensetzung nach Anspruch 2, **dadurch gekennzeichnet, dass** der Gehalt an dem Ginsenosid Rg1 50-65 Gew.-%, an dem Ginsenosid Re 6-9 Gew.-% und an dem Notoginsenosid R1 10~15 Gew.-% beträgt.

4. Panaxatriol-Saponine-Zusammensetzung nach Anspruch 3, **dadurch gekennzeichnet, dass** der Gehalt an dem Ginsenosid Rg1 55-65 Gew.-% beträgt.

5. Panaxatriol-Saponine-Zusammensetzung nach einem beliebigen der Ansprüche 1-4, wobei die Dosierungsformen der Panaxatriol-Saponine-Zusammensetzung Granulat, Gemisch, Tablette, Arzneipulver, Honigpille, Kapsel, Injektion, Tropfen oder Pflaster enthalten.

6. Verfahren zum Bereiten einer Panaxatriol-Saponine-Zusammensetzung nach Anspruch 1, umfassend die folgenden Schritte:
(1) Einlegen einer Radix Notoginseng in einen Ethylalkohol von 50-90 Gew.-%, um eine Panaxatriol-Saponine-Zusammensetzung zu extrahieren, wobei das Gewicht des verwendeten Ethylalkohols mindestens 3-mal schwerer ist als das der Radix Notoginseng, dann Entnehmen der Ethylalkohol-Extraktionslösung der Radix Notoginseng;
(2) Zufügen der Ethylalkohol-Extraktionslösung zu einer Chromatographiesäule aus makroporösen Absorptionsharzen des Polystyroltyps; und
(3) Eluieren der Chromatographiesäule aus den makroporösen Absorptionsharzen des Polystyroltyps mit einem Ethylalkohol von 30-50 Gew.-% und Entnehmen des Eluenten.

7. Verfahren zum Bereiten einer Panaxatriol-Saponine-Zusammensetzung nach Anspruch 6, **dadurch gekennzeichnet, dass** der Schritt (1) wie folgt ausgeführt wird:
(a) Pulverisieren der Radix Notoginseng, um Radix-Notoginseng-Pulver zu erhalten;
(b) Einlegen des in Schritt (a) erhaltenen Radix-Notoginseng-Pulvers in einen Ethylalkohol von 50-90 Gew.-%, wobei das Gewicht des verwendeten Ethylalkohols mindestens 2-mal schwerer ist als das des Radix-Notoginseng-Pulvers; und
(c) Perkolieren des in Schritt (b) erhaltenen Radix-Notoginseng-Pulvers mit einem Ethylalkohol von 50-90 Gew.-%, wobei das Gewicht des verwendeten Ethylalkohols mindestens 1-mal schwerer ist als das des Radix-Notoginseng-Pulvers; dann Entnehmen der Perkolationsflüssigkeit.

8. Verfahren zum Bereiten einer Panaxatriol-Saponine-Zusammensetzung nach Anspruch 7, **dadurch gekennzeichnet, dass** die Konzentration des in den Schritten (b) und (c) verwendeten Ethylalkohols 55-70 Gew.-% beträgt.

9. Verfahren zum Bereiten einer Panaxatriol-Saponine-Zusammensetzung nach Anspruch 8, **dadurch gekennzeichnet, dass** die Konzentration des in den Schritten (b) und (c) verwendeten Ethylalkohols ungefähr 60 Gew.-% beträgt.

10. Verfahren zum Bereiten einer Panaxatriol-Saponine-Zusammensetzung nach Anspruch 6, **dadurch gekennzeichnet, dass** die Konzentration des in Schritt (3) zum Eluieren des Harzes verwendeten Ethylalkohols 35-45 Gew.-% beträgt, wobei das Gewicht des Ethylalkohols zur Verwendung mindestens 1-mal schwerer ist als dasjenige des Radix-Notoginseng-Pulvers.

11. Verfahren zum Bereiten einer Panaxatriol-Saponine-Zusammensetzung nach Anspruch 10, **dadurch gekennzeichnet, dass** die Konzentration des in Schritt (3) zum Eluieren des Harzes verwendeten Ethylalkohols 40 Gew.-% beträgt.

12. Verfahren zum Bereiten einer Panaxatriol-Saponine-Zusammensetzung nach einem beliebigen der Ansprüche 6-11, **dadurch gekennzeichnet, dass** es weiter den folgenden Schritt enthält: Konzentrieren des in Schritt (3) entnommenen Eluenten, um die Extrakte der Panaxatriol-Saponine-Zusammensetzung zu erhalten.

13. Verfahren zum Bereiten einer Panaxatriol-Saponine-Zusammensetzung nach einem beliebigen der Ansprüche 6-11, **dadurch gekennzeichnet, dass** es weiter den folgenden Schritt enthält: Aufbereiten des in Schritt (3) erhaltenen Eluenten durch Säulenchromatographie oder Lösemittel-Extraktion.

14. Verfahren zum Bereiten einer Panaxatriol-Saponine-Zusammensetzung nach Anspruch 12, **dadurch gekennzeichnet, dass** es weiter den Schritt des Trocknens der Panaxatriol-Saponine-Extrakte umfasst.

15. Verwendung der Panaxatriol-Saponine-Zusammensetzung nach einem beliebigen der Ansprüche 1-4 zum Bereiten einer Arznei zum Behandeln der vaskulären Thromboseerkrankung.

16. Panaxatriol-Saponine-Zusammensetzung nach Anspruch 1 bis 4 zur Verwendung bei einem Verfahren zum Behandeln der vaskulären Thromboseerkrankung, wobei das Verfahren die Verabreichung einer wirksamen Dosierung der Panaxatriol-Saponine-Zusammensetzung an Patienten umfasst, die unter vaskulärer Thromboseerkrankung leiden.

## Revendications

1. Composition de saponines de panaxatriol obtenue par extraction et purification du Notoginseng Radix, contenant essentiellement un ginsénoside Rg1, un ginsénoside Re et un notoginsénoside R1, dans laquelle la teneur du ginsénoside Rg1 est de 30-70 % en poids, celle du ginsénoside Re est de 4~15 % en poids, celle du notoginsénoside R1 est de 7-25 % en poids et la somme maximale des teneurs du ginsénoside Rg1, du Re et du notoginsénoside R1 est de 98 % en poids.

2. Composition de saponines de panaxatriol selon la revendication 1, **caractérisée en ce que** la teneur du ginsénoside Rg1 est de 40-65 % en poids, celle du ginsénoside Re est de 4~10 % en poids et celle du notoginsénoside R1 est de 8~18 % en poids.

3. Composition de saponines de panaxatriol selon la revendication 2, **caractérisée en ce que** la teneur du ginsénoside Rg1 est de 50-65 % en poids, celle du ginsénoside Re est de 6-9 % en poids et celle du notoginsénoside R1 est de 10~15 % en poids.

4. Composition de saponines de panaxatriol selon la revendication 3, **caractérisée en ce que** la teneur du ginsénoside Rg1 est de 55-65 % en poids.

5. Composition de saponines de panaxatriol selon l'une quelconque des revendications 1-4, dans laquelle les formes pharmaceutiques de la composition de saponines de panaxatriol comprennent les granulés, le mélange, le comprimé, la poudre médicinale, la pilule de miel, la capsule, l'injection, les gouttes ou l'emplâtre.

6. Méthode pour préparer une composition de saponines de panaxatriol selon la revendication 1, comprenant les étapes suivantes :
(1) immerger un Notoginseng Radix dans un alcool éthylique de 50-90 % en poids afin d'extraire une composition de saponines de panaxatriol, sachant que le poids de l'alcool éthylique utilisé est au moins trois fois plus lourd que celui du Notoginseng Radix, puis collecter la solution d'extraction à l'alcool éthylique du Notoginseng Radix ;
(2) ajouter la solution d'extraction à l'alcool éthylique dans une colonne chromatographique de résines absorbantes macroporeuses de type polystyrène ; et
(3) éluer la colonne chromatographique de résines absorbantes macroporeuses de type polystyrène avec un alcool éthylique de 30-50 % en poids et collecter l'éluant.

7. Méthode pour préparer une composition de saponines de panaxatriol selon la revendication 6, **caractérisée en ce que** l'étape (1) est accomplie comme suit :
(a) pulvériser le Notoginseng Radix afin d'obtenir la poudre de Notoginseng Radix ;
(b) immerger la poudre de Notoginseng Radix obtenue à l'étape (a) dans un alcool éthylique de 50-90 % en poids, sachant que le poids de l'alcool éthylique utilisé est au moins deux fois plus lourd que celui de la poudre de Notoginseng Radix ; et
(c) percoler la poudre de Notoginseng Radix obtenue à l'étape (b) dans un alcool éthylique de 50-90 % en poids, sachant que le poids de l'alcool éthylique utilisé est au moins une fois plus lourd que celui de la poudre de Notoginseng Radix ; puis collecter le liquide percolateur.

8. Méthode pour préparer une composition de saponines de panaxatriol selon la revendication 7, **caractérisée en ce que** la concentration de l'alcool éthylique utilisé aux étapes (b) et (c) est de 55-70 % en poids.

9. Méthode pour préparer une composition de saponines de panaxatriol selon la revendication 8, **caractérisée en ce que** la concentration de l'alcool éthylique utilisé aux étapes (b) et (c) est de 60 % en poids environ.

10. Méthode pour préparer une composition de saponines de panaxatriol selon la revendication 6, **caractérisée en ce que** la concentration de l'alcool éthylique utilisé à l'étape (3) pour éluer la résine est de 35-45 % en poids, sachant que le poids de l'alcool éthylique utilisé est au moins une fois plus lourd que celui de la poudre de Notoginseng Radix.

11. Méthode pour préparer une composition de saponines de panaxatriol selon la revendication 10, **caractérisée en ce que** la concentration de l'alcool éthylique utilisé à l'étape (3) pour éluer la résine est de 40 % en poids environ.

12. Méthode pour préparer une composition de saponines de panaxatriol selon l'une quelconque des revendications 6-11, caractérisée en ce qu'elle comprend en outre l'étape suivante : concentrer l'éluant collecté à l'étape (3) afin d'obtenir les extraits de la composition de saponines de panaxatriol.

13. Méthode pour préparer une composition de saponines de panaxatriol selon l'une quelconque des revendications 6-11, caractérisée en ce qu'elle comprend en outre l'étape suivante : purifier l'éluant obtenu à l'étape (3) par chromatographie sur colonne ou extraction par solvant.

14. Méthode pour préparer une composition de saponines de panaxatriol selon la revendication 12, **caractérisée en ce qu'**elle comprend en outre l'étape consistant à sécher les extraits de saponines de panaxatriol.

15. Utilisation de la composition de saponines de panaxatriol selon l'une quelconque des revendications 1-4 pour préparer le médicament pour traiter la maladie du thrombus vasculaire.

16. Composition de saponines de panaxatriol selon la revendication 1 à 4 pour l'utilisation dans une méthode pour traiter la maladie du thrombus vasculaire, sachant que ladite méthode comprend l'administration d'une dose efficace de ladite composition de saponines de panaxatriol à des patients souffrant de la maladie du thrombus vasculaire.
